# FASCICULE DE BREVET EUROPEEN

(11) **EP 3 651 707 B1**
(45) Date de publication et mention de la délivrance du brevet: **27.04.2022**
(21) Numéro de dépôt: 18749037.0
(22) Date de dépôt: 12.07.2018
(51) Int. Cl.: A61F 13/00, A61L 15/00, C08L 53/00

(54) **COMPOSITION POUR PANSEMENT INTERFACE**
ZUSAMMENSETZUNG FÜR SCHNITTSTELLENVERBAND
COMPOSITION FOR INTERFACE DRESSING

(30) Priorité: 12.07.2017 FR 1756589
(43) Date de publication de la demande: 20.05.2020
(73) Titulaire: Urgo Recherche Innovation et Développement, 21300 Chenove (FR)
(72) Inventeur: MEYER, Nadège, 21110 Tart le Haut (FR)
(74) Mandataire: Plasseraud IP
(86) Numéro de dépôt international: PCT/FR2018/051759
(87) Numéro de publication internationale: WO 2019/012230

(56) Documents cités:
- WO-A1-2016/097653
- US-A1- 2002 128 345
- US-A1- 2010 285 129
- US-A1- 2015 174 285

## Description

### RESUME

La présente invention selon les revendications 1 à 10 est relative à une nouvelle composition à base de copolymères tribloc du type ABA, comportant deux blocs terminaux thermoplastiques A styrène et une séquence centrale élastomère B qui est une oléfine saturée, et d'au moins une résine choisie parmi les résines aromatiques, utilisable notamment pour la réalisation d'un pansement interface comprenant une armature ou un support, ou un pansement interface autoporté, de préférence autoporté.

La présente invention est également relative à un pansement interface autoporté qui présente une manipulabilité facilitée car il présente une bonne résistance à la déchirure.

### ETAT DE LA TECHNIQUE ANTERIEURE

On connaît depuis longtemps le traitement des plaies par des pansements désignés par le terme « pansements interface », destinés à être mis au contact de la plaie en assurant une interface entre la plaie et une compresse absorbante que l'on dépose sur le pansement pour absorber les exsudats.

Le pansement URGOTUL^{®}commercialisé depuis 2000 par la société Laboratoires URGO est un exemple illustratif de pansement interface. Le produit URGOTUL^{®} est constitué d'une armature faite d'un tissu à mailles ouvertes dont les fils sont enrobés d'un gel cohésif, de façon à laisser les mailles essentiellement non obturées. Ce gel est formé d'une composition constituée d'une matrice élastomérique à base de copolymères tribloc du type ABA (styrène-oléfine saturée-styrène) fortement plastifiée et contenant en dispersion une faible quantité de particules hydrophiles d'un hydrocolloïde. Ce pansement et sa composition sont décrits dans l'exemple 1 de la demande de brevet WO 00/16725. La composition qualitative et quantitative de la matrice élastomérique de ce pansement lui confère des propriétés remarquables en ce qui concerne la favorisation du processus de cicatrisation et en particulier de la prolifération des fibroblastes.

Le produit URGOTUL^{®} présente néanmoins l'inconvénient, dans les cas où l'on souhaite l'appliquer sur des plaies difficilement recouvrables, par exemple en raison de leur localisation, de manquer de conformabilité, à cause de la rigidité de son armature.

Pour résoudre ce problème, des pansements interface autoportés (sans armature) ont été décrits dans la demande de brevet FR 2 936 158. Les produits décrits dans cette demande présentent à la fois une bonne élasticité et une cohésion suffisante pour pouvoir être manipulés. Toutefois, les pansements interface autoportés décrits dans ce document mettent en œuvre des constituants différents des polymères tribloc du type ABA employés dans le pansement URGOTUL^{®} de sorte que les propriétés de cicatrisation et de prolifération des fibroblastes ne sont pas préservées.

En outre, dans une logique de rentabilité économique, il serait souhaitable pour la demanderesse de pouvoir fabriquer un pansement interface autoporté à partir de composés de même nature, voire identiques, à ceux utilisées pour la fabrication du pansement URGOTUL^{®} et ses déclinaisons, qu'elle produit déjà.

Outre l'aspect économique, ceci présenterait aussi un avantage non négligeable dans le cas où l'on souhaite incorporer dans la composition du pansement interface autoporté des composés actifs, c'est-à-dire, des composés qui ont une action sur le processus de cicatrisation ou le traitement de la plaie, comme par exemple des antibactériens, tels que les sels d'argent, ou des inhibiteurs de MMP (Matrix métalloprotéases) comme le sel de potassium de sucrose octasulfate. En effet, l'incorporation d'actifs dans ce type de composition est toujours délicate et complexe, chaque composé de la composition pouvant interagir avec les autres, modifier les propriétés rhéologiques et physico chimiques de la composition, ou encore affecter la stabilité ou la solubilité de l'actif.

En utilisant des composés de même nature que ceux déjà utilisés pour la fabrication du pansement URGOTUL^{®}, les chances de mettre au point de nouvelles compositions présentant les avantages recherchés sont donc optimisées.

Enfin, l'utilisation de composés de même nature voire identiques à ceux utilisés pour la fabrication du pansement URGOTUL^{®} permettrait de préserver les propriétés remarquables du produit URGOTUL^{®} sur la prolifération des fibroblastes et sur le processus de cicatrisation.

Il serait donc souhaitable de disposer d'une composition pour pansement interface constituée de composés de même nature ou identiques à ceux employés dans le produit URGOTUL^{®} permettant de préparer des pansements interface présentant une bonne conformabilité et une bonne résistance à la déchirure, même sans armature.

### RESUME DE L'INVENTION

Ainsi, la présente invention a permis de mettre au point une composition spécifique à base de copolymères tribloc du type ABA, comportant deux blocs terminaux thermoplastiques A styrène et une séquence centrale élastomère B qui est une oléfine saturée, permettant la préparation d'une matrice élastomérique suceptible d'être mise en œuvre dans un pansement, qui, de préférence, est autoporté, ladite matrice présentant une très bonne résistance à la déchirure.

Plus précisément, il a été découvert, et ceci constitue le fondement de la présente invention, que des compositions comportant au moins deux copolymères (élastomères) tribloc spécifiques du type styrène - oléfine saturée - styrène, et au moins une résine aromatique présents au sein de la composition en une quantité pondérale prédéterminée, permettent de réaliser des pansements qui présentent des propriétés mécaniques améliorées. En effet, les pansements obtenus avec les matrices élastomériques de l'invention se manipulent facilement, et le risque de déchirure au moment de l'application ou au moment du retrait du pansement par le personnel soignant est diminué. Une fois appliqués, les pansements obtenus avec ces matrices élastomériques présentent une conformabilité améliorée, le contact avec le lit de la plaie est par conséquent favorisé. Du fait de son élasticité améliorée, le pansement suit mieux les mouvements du patient, et le renforcement de ses propriétés mécaniques permet d'éviter que le pansement ne se délite dans la plaie, même après contact avec les exsudats de la plaie sur une durée prolongée.

L'ajout de résines spécifiques permet de diminuer la viscosité du mélange élastomérique et de renforcer mécaniquement la matrice élastomérique. Ceci est particulièrement avantageux dans le procédé de fabrication de la matrice élastomérique. La composition, du fait de sa viscosité diminuée, peut être enduite plus facilement sur une armature. Dans le cas d'un pansement autoporté, l'amélioration de la cohésion de la matrice élastomérique permet un meilleur moulage et démoulage de cette dernière. En outre, du fait des résines spécifiques utilisées, la température de fabrication de la composition peut être diminuée d'environ 10°C, ce qui permet d'introduire dans le mélange des composants sensibles aux traitements thermiques, tels que des actifs par exemple. Selon un mode de réalisation préféré, le pansement selon l'invention n'adhère pas aux gants chirurgicaux en latex.

Des compositions à base d'élastomères pour le relargage d'actifs ont déjà été proposées par exemple dans le document US2002/0128345. Ce document décrit des compositions adhésives sensibles à la pression, mettant en œuvre des copolymères triblocs de viscosité élevée en association avec des résines tackifiantes conférant des propriétés de collant à la composition. En particulier, les compositions comprennent de préférence un copolymère dibloc de viscosité particulièrement élevée. Les matrices obtenues au moyen des compositions décrites dans le document US2002/0128345 présentent ainsi des propriétés d'adhérence à la peau particulièrement élevées. De telles compositions ne conviennent toutefois pas à la mise en œuvre de pansements interface autoportés, ayant vocation à être mis en contact avec une plaie à laquelle il est impératif qu'ils n'adhèrent pas pour permettre un retrait atraumatique du pansement. De tels pansements interfaces sont généralement utilisés en association avec un dispositif de maintien, tel qu'une bande (également appelé pansement secondaire). En outre, des compositions adhésives selon US2002/0128345 ne présentent pas la manipulabilité souhaitée pour de tels pansements. Enfin, le collant conféré à de telles compositions altère de manière inhérente la cohésion des matrices (on entend par cohésion le comportement mécanique dans le cadre d'un pansement interface autoporté) obtenues à partir de ces compositions. En effet, les composition décrites dans ce document sont particulièrement visqueuses et emprisonnent des bulles d'air dans leur structure lors du procédé de fabrication. Ainsi, ces bulles s'intègrent à la structure des matrices obtenues à partir de telles composition, fragilisant leur structure. US 2010/285129 A, US 2002/128345 A1 et WO 2016/097653 A1 décrivent des compositions permettant la libération contrôlée et prolongée dans des pansements et comprenant des triblocs copolymères, des plastifiants et un actif.

Ainsi selon un premier aspect, la présente invention a pour objet une composition, notamment utile pour la fabrication de pansements, comprenant:
- 5 à 20% d'un mélange de 2 copolymères tribloc du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0 ,01 et 0,5 Pa.s mesurée à 30°C dans une solution à 15 % (masse / masse) dans le toluène
- 50 à 80% en poids d'au moins un plastifiant,
- 5 à 20% d'au moins une résine de type alpha-méthyl styrène dont le point de ramollissement se situe entre 80 et 125°C, de préférence entre 90 et 110°C, étant entendu que :
- lorsque la teneur en copolymères tribloc est comprise entre 5 et 10% en poids, la teneur en résine est comprise entre 15 et 20% en poids, rapporté au poids total de la composition et
- lorsque la teneur en copolymères tribloc est supérieure à 10% en poids, la teneur en résine est comprise entre 5 et 20% en poids, rapporté au poids total de la composition,

les pourcentages étant rapportés au poids total de la composition.

Selon un second aspect, la présente invention a pour objet une matrice élastomérique obtenue à partir d'une telle composition et un pansement interface, avec support ou autoporté comprenant ladite matrice élastomérique.

### DESCRIPTION DETAILLEE

### Elastomère

La composition selon l'invention, comprend un mélange de 2 copolymères tribloc du type ABA.

En particulier, ce mélange de deux copolymères comprendra au moins un copolymère qui présente une viscosité comprise entre 0,01 et 1 Pa.s mesurée à 30°C dans une solution à 5 % masse/masse dans le toluène et au moins un copolymère présentant une viscosité comprise entre 0,01 et 0,5 Pa.s mesurée à 30°C dans une solution à 15% (masse/masse) dans le toluène.

Les copolymères séquencés utilisés dans le cadre de l'invention sont des copolymères triblocs du type ABA comportant deux blocs terminaux thermoplastiques A styrène et une séquence centrale élastomère B qui est une oléfine saturée. Les séquences B d'oléfines saturées sont par exemple des séquences éthylène-butylène, éthylène-propylène ou éthylène-éthylène-propylène.

Par souci de simplicité, dans la présente description, les blocs polymériques constituant les copolymères précités sont désignés par la nature de leurs unités récurrente. Ainsi, l'expression « bloc » ou « séquence styrène A » désigne une séquence poly(styrène) et l'expression « bloc » ou « séquence oléfine saturée » désigne une séquence poly(oléfine saturée).

Les copolymères triblocs à séquence centrale saturée sont bien connus de l'homme de l'art et sont par exemple commercialisés :
- par la société KRATON sous la dénomination KRATON^{®} G, et en particulier les grades KRATON^{®} G1651, KRATON^{®} G1654, KRATON^{®} G 1657, KRATON^{®} G1652 ou KRATON^{®} G1650 et par la société KURARAY sous les dénominations SEPTON^{®} et en particulier les grades 8006 ou 8004 pour les copolymères séquencés poly (styrène-éthylène-butylène-styrène) (en abrégé SEBS);
- par la société KURARAY sous la dénomination SEPTON^{®} pour les copolymères séquencés poly (styrène-éthylène-propylène-styrène) (en abrégé SEPS) et en particulier les grades 2005,2006 ou 2063 et pour les polymères séquencés poly (styrène-éthylène-éthylène-propylène-styrène) (en abrégé SEEPS) et en particulier les grades 4033,4044, 4055, 4077 ou 4099.

Dans le cadre de la présente invention, on préférera les copolymères triblocs SEBS, SEPS ou SEEPS ayant une teneur en styrène comprise entre 25 et 45 % en poids par rapport au poids dudit copolymère SEBS, SEPS ou SEEPS.

Parmi les copolymères qui présentent une viscosité comprise entre 0,01 et 1 Pa.s mesurée dans une solution à 5% (masse/masse) dans le toluène, on peut citer les copolymères commercialisé par la société KRATON sous les grades KRATON^{®} G 1651 et KRATON^{®} G 1654 et les copolymères commercialisés par la société KURARAY sous les grades SEPTON^{®} 2005, 2006, 8006, 4055, 4077, 4044 ou 4099.

Parmi les copolymères qui présentent une viscosité comprise entre 0,01 et 0,5 Pa.s mesurée dans une solution à 15% (masse/masse) dans le toluène on peut citer les copolymères commercialisés par la société KRATON sous les grades KRATON^{®} G 1650, KRATON^{®} G 1657 et KRATON^{®} G 1652 et les copolymères commercialisés par la société KURARAY sous les grades SEPTON^{®} 2063 ou 4033.

Ces viscosités sont mesurées à 30°C à l'aide d'un viscosimètre Brookfield modèle LVI dans une solution dans le toluène à 5 % ou 15 % masse/masse en fonction du poids moléculaire du copolymère. En particulier, viscosités sont mesurées selon la norme ISO2555.

De façon générale, la quantité de copolymères dans la composition finale pourra être comprise entre 5 et 20 % en poids, de préférence entre 7 et 15 % en poids, rapportée au poids total de la composition.

Dans le cadre de la présente invention on préférera tout particulièrement l'utilisation de deux copolymères séquencés SEBS, et en particulier l'association des copolymères KRATON^{®} G 1654 et KRATON^{®} G 1650 dans laquelle le KRATON^{®} G 1654 est présent en une quantité de 5 à 10% en poids, rapportée au poids total de la composition et le KRATON^{®} G 1650 est présent en une quantité de 2 à 5% en poids, rapportée au poids total de la composition.

De préférence, ce mélange de deux copolymères comprendra donc au moins 5 à 10% en poids d'un copolymère qui présente une viscosité comprise entre 0,01 et 1 Pa.s mesurée dans une solution à 5 % masse/masse dans le toluène et au moins 2 à 5% d'un copolymère présentant une viscosité comprise entre 0,01 et 0,5 Pa.s mesurée dans une solution à 15% (masse/masse) dans le toluène, rapporté au poids total de la composition.

En particulier, les compositions selon l'invention ne comprennent pas de polymère dibloc, en particulier ne comprennent pas de polymère dibloc styrène isoprène hydrogéné.

### Les résines

Les résines mises en œuvre dans la composition selon invention sont des résines hydrocarbonées aromatiques, c'est-à-dire à base de monomères aromatiques uniquement. Elles se distinguent des résines aliphatiques, à base de monomères aliphatiques uniquement, ou des résines aliphatiques/aromatiques à base de monomères aliphatiques et aromatiques. Sans vouloir être lié par une quelconque théorie, il semble que ces résines présentent une bonne solubilité dans le bloc A des copolymères ABA et renforcent ce bloc styrène, ce qui améliore la cohésion de la matrice élastomérique finale obtenue.

En particulier, le monomère aromatique est l'alpha-méthyl styrène. Ainsi, selon un mode de réalisation particulièrement préférentiel, la résine hydrocarbonée aromatique est choisie parmi les résines d'homopolymères et copolymères d'alpha-méthyl-styrène.

Parmi les résines aromatiques testées, un certain nombre d'entre elles ne donnaient pas entière satisfaction. En effet, certains grades de résine, du fait de leur point de ramolissement élevé, nécessitent d'être chauffées à des températures élevées (au-delà de 140°C) pour réaliser la composition de l'invention. En travaillant à de telles températures, il existe un risque d'évaporation du plastifiant. Lorsque l'on ajoute des hydrocolloïdes (tels que la carboxymethylcellulose) ou des actifs dans la composition, ceux-ci risquent de se dégrader.

Ainsi, les résines mises en œuvre dans les compositions selon l'invention sont les résines de type alpha-méthyl styrène dont le point de ramollissement se situe entre 80 et 125°C, de préférence entre 90 et 110°C.

Ces résines donnent les meilleurs résultats en termes de procédé de fabrication, de force à la rupture et d'allongement à la rupture. L'ajout de telles résines permet de diminuer la viscosité du mélange et de renforcer mécaniquement la composition, facilitant ainsi sa transformation en une matrice polymérique résistante à la déchirure.

Le point de ramollissement est mesuré selon la norme ISO 4625 (méthode "Ring and Bail").

De préférence, la résine selon l'invention est une résine alpha-méthyl styrène présentant un point de ramollissement situé entre 95 et 105°C ou entre 115 et 125°C ou une résine poly(styrène-co-alpha-méthyl styrène) présentant un point de ramollissement entre 95°C et 115°C.

Les résines préférentielles ci-dessus sont bien connues de l'homme du métier et disponibles commercialement, par exemple vendues sous les dénominations commerciales suivantes :
- Sylvares SA 100 et Sylvares SA 120 d'Arizona Chemical : résines alpha-méthyl styrène présentant un point de ramollissement situé entre 95 et 105°C ou entre 115 et 125°C respectivement,
- la résine Cleartack W90 ou Norsolène W90 de Cray Valley : résine poly(styrene-co-alpha-methylstyrene) présentant un point de ramollissement entre 85 et 95°C, ,
- les résines Kristalex 3100LV, Kristalex F100, Kristalex 3105SD et Kristalex F115 d'Eastman : résines poly(styrene-co-alpha-methylstyrene) présentant un point de ramollissement de 100°C, ou entre 96 et 104°C ou de 105°C, ou entre 114 et 120°C respectivement.

Selon un mode préféré de réalisation, les compositions selon l'invention ne comprennent pas de résine autre que la alpha-méthyl styrène précédemment décrite, et en particulier, ne comprennent pas de résine tackifiante ou collante c'est-à-dire des résines qui apportent un caractère adhérent à la matrice élastomérique de manière permanente à température constante.

Dans le cadre de la présente invention, la résine est de préférence présente en une quantité de 5 à 20%, de préférence 5 à 15% en poids, rapportée au poids total de la composition.

Dans le cadre de la présente invention :
- lorsque la teneur en copolymères tribloc est comprise entre 5 et 10% en poids, la teneur en résine est comprise entre 15 et 20% en poids, rapporté au poids total de la composition et
- lorsque la teneur en copolymères tribloc est supérieure à 10% en poids, la teneur en résine est comprise entre 5 et 20% en poids, rapporté au poids total de la composition,

les pourcentages étant rapportés au poids total de la composition.

Dans le cadre de la présente invention, la teneur en copolymères tribloc et en résine représente 16 à 40% en poids, de préférence de 20 à 35% en poids, rapporté au poids total de la composition.

### Le plastifiant

Afin de réaliser des pansements interface, le mélange de copolymères et la résine présents dans la composition selon l'invention sont associés à un (ou plusieurs) composé(s) plastifiants.

Les plastifiants susceptibles d'être utilisés sont bien connus et destinés à améliorer les propriétés d'étirement, de souplesse, d'extrudabilité ou de mise en œuvre des copolymères. On pourra utiliser à cet effet un ou plusieurs plastifiants si nécessaire.

D'une façon générale, en tant que plastifiants, on préférera des composés liquides, compatibles avec la séquence centrale oléfine saturée des copolymères séquencés précités.

Parmi les composés plastifiants susceptibles d'être utilisés à cet effet, on citera en particulier les huiles minérales plastifiantes.

Alternativement, on peut aussi utiliser des produits de synthèse à base de mélanges liquides d'hydrocarbures saturés comme par exemple les produits commercialisés par la société TOTAL sous la dénomination GEMSEAL^{®} et en particulier le produit GEMSEAL^{®} 60 qui est un mélange isoparaffinique issu d'une coupe pétrolière totalement hydrogénée.

Dans le cadre de la présente invention, on utilisera de préférence des huiles plastifiantes et en particulier des huiles minérales formées de composés de nature paraffinique ou naphténique, ou de leurs mélanges, dans des proportions variables.

Des huiles minérales plastifiantes particulièrement préférées sont formées de mélanges de composés de nature paraffinique et naphténique, et en particulier de tels mélanges dans lesquels la proportion de composés de nature paraffinique est majoritaire.

Parmi les huiles plastifiantes convenant particulièrement, on peut citer les produits commercialisés par la société SHELL sous les dénominations ONDINA^{®} et en particulier ONDINA^{®} 919 ou l'huile commercialisée par la société PETRO CANADA sous la référence PURETOL^{®} 9D ou l'huile BLANDOL commercialisée par Sonneborn ou encore l'huile Pionier 2076P commercialisé par Hansen & Rosenthal.

Outre des huiles, le plastifiant peut comprendre de la vaseline. La vaseline mise en œuvre dans les compositions de l'invention est une vaseline conforme à la Pharmacopée Française disponible commercialement.

Dans le cadre de la présente invention, la vaseline est présente en une quantité de 1 à 30%, de préférence 5 à 25% en poids, rapportée au poids total de la composition.

Dans le cadre de la présente invention, le plastifiant est présent en une quantité de 50 à 80%, de préférence 60 à 70% en poids, rapportée au poids total de la composition.

De préférence, le plastifiant est constitué d'un mélange d'huile minérale et de vaseline, l'huile minérale étant présente en une quantité allant de 45 à 60% en poids rapporté au poids total de la composition, la vaseline étant présente en une quantité allant de 5 à 20% en poids rapporté au poids total de la composition.

Sur la base des tests décrits dans les exemples de la présente demande, une trame élastomérique présentant une force à la rupture de 1,5 à 2,5 N/cm et un allongement à la rupture de 640% à 900%, de préférence 700% à 900% présentera les meilleures propriétés mécaniques. En comparaison avec une formulation identique sans résine, on améliore d'au moins 40% la force à la rupture.

Pour obtenir ce résultat il a été déterminé que la composition selon l'invention comprend de préférence:
- 5 à 20% en poids d'un mélange de deux copolymères comprenant au moins un copolymère qui présente une viscosité comprise entre 0,01 et 1 Pa.s mesurée dans une solution à 5 % masse/masse dans le toluène et au moins un copolymère présentant une viscosité comprise entre 0,01 et 0,5 Pa.s mesurée dans une solution à 15% (masse/masse) dans le toluène.
- 50 à 80% en poids d'un mélange d'huile et de vaseline,
- 5 à 20 % en poids d'une résine choisie parmi les résines alpha-méthyl styrène présentant un point de ramollissement situé entre 95 et 105°C ou entre 115 et 125°C ou une résine poly(styrène-co-alpha-méthyl styrène) présentant un point de ramollissement entre 95°C et 115°C.

Ces compositions sont également utiles pour la préparation de pansements avec ou sans armature.

### Les hydrocolloïdes

Selon un mode de réalisation de l'invention particulièrement préféré dans le cadre de la réalisation de pansements interface autoportés, avec support ou avec armature pour la cicatrisation des plaies, les compositions selon l'invention comprennent des particules hydrophiles d'un hydrocolloïde (ou particules d'hydrocolloïde).

Ces particules permettent en effet le retrait indolore d'un pansement interface et le maintien d'un milieu humide au niveau de la plaie afin de favoriser la cicatrisation.

A cet effet, une quantité faible de particules hydrophiles d'un hydrocolloïde est ainsi soit disposée en surface de la matrice élastomérique une fois celle-ci formée soit, de préférence, dispersée de façon homogène au sein de la composition selon l'invention.

Par « hydrocolloïde » ou « particules d'hydrocolloïde », on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium, ainsi que les polymères synthétiques à base de sels de l'acide acrylique, connus sous l'appellation "superabsorbants", comme par exemple les produits commercialisés par la société CIBA Specialty Chemicals sous la dénomination SALCARE^{®} SC91 ainsi que les mélanges de ces composés.

Certains de ces superabsorbants qualifiés de « microcolloïdes » car ils présentent une taille de particules inférieure à 10 micromètres peuvent bien entendu être également utilisés.

Les hydrocolloïdes préférés dans le cadre de la présente invention sont les sels de métal alcalin de la carboxyméthylcellulose, et en particulier la carboxyméthylcellulose de sodium (CMC).

La taille des particules d'hydrocolloïde est généralement comprise entre 50 et 100 microns, avantageusement de l'ordre de 80 microns.

D'une façon générale, la quantité de particules d'hydrocolloïde incorporées dans la composition selon l'invention sera avantageusement inférieure ou égale à 25 % en poids, avantageusement de l'ordre de 2 à 20 % en poids, de préférence de 5 à 18 % en poids, de préférence encore de 10 à 15 % en poids, rapportée au poids total de ladite composition.

Si les particules d'hydrocolloïde sont disposées en surface de la matrice élastomérique un fois celle-ci formée, leur quantité sera de préférence de l'ordre de 1 à 10 % et plus particulièrement de 2 à 5 % en poids, rapportée au poids total de ladite matrice élastomérique.

Le choix d'une quantité de particules d'hydrocolloïde comprise dans ces plages de valeurs est importante pour la réalisation d'un pansement interface, et en particulier un pansement interface autoporté aéré, afin d'éviter que la gélification de la composition n'entraine la fermeture des trous traversants lors de l'absorption des exsudats.

### Les antioxydants

La composition selon l'invention peut également comprendre des agents antioxydants.

Par « agents antioxydants », on entend désigner ici les composés couramment employés par l'homme de l'art pour assurer la stabilité des composés entrant dans la formulation des compositions, en particulier vis-à-vis de l'oxygène, la chaleur, l'ozone ou les rayonnements ultra-violets.

Comme exemples d'agents antioxydants appropriés, on peut citer notamment les antioxydants phénoliques comme en particulier les produits commercialisés par la société BASF sous les dénominations IRGANOX^{®} 1010, IRGANOX^{®} 565, IRGANOX^{®} 1076.

D'une façon générale ces agents antioxydants pourront être utilisés seuls ou en association en une quantité de l'ordre de 0,05 à 1 % en poids, de préférence de 0,05 à 0,2 % en poids, rapportée au poids total de la composition.

Dans le cadre de la présente invention, on préférera l'utilisation du produit IRGANOX^{®} 1010 en une quantité comprise entre 0,05 et 0 ,2 % en poids, rapportée au poids total de la composition.

### Actifs additionnels

Outre les agents antioxydants, la composition selon l'invention peut comprendre une (ou plusieurs) autre(s) substance(s) active(s) permettant d'induire ou d'accélérer la cicatrisation ou pouvant avoir un rôle favorable dans le traitement d'une plaie.

Parmi ces substances actives, on peut citer, en particulier, à titre d'exemples:
- les agents favorisant la cicatrisation tels que le rétinol, la vitamine A, la vitamine E, la N-Acétyl Hydroxyproline, les extraits de Centella Asiatica, la papaïne, la silicone, les huiles essentielles de thym, niaouli, romarin, sauge, l'acide hyaluronique, le sucrose octasulfate de potassium, le sucralfate, l'allantoïne, la metformine
- les agents antibactériens tels que les sels ou complexes d'argent (tels les sulfates d'argent, les nitrates d'argent, les sulfamides d'argent ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, les probiotiques ;
- les antiseptiques tels que la chlorhexidine, le trichlosan, le biguanide, l'hexamidine, le thymol, le lugol, la povidone iodée, le chlorure de benzalkonium et de benzethonium ;
- les anti-douleurs tels que le paracétamol, la codéine, le dextropropoxyphène, le tramadol, la morphine et ses dérivés, les corticoïdes et leurs dérivés ;
- les anesthésiques locaux tels que la lidocaïne, la benzocaïne, la dibucaïne, le chlorhydrate de pramoxine, la bupivacaïne, la mépivacaïne, la prilocaïne, l'étidocaïne ;
- les anti-inflammatoires comme les anti-inflammatoires non stéroïdiens (AINS), l'aspirine ou acide acétylsalicylique, l'ibuprofène, le kétoprofène, le flurbiprofène, le diclofenac, l'acéclophénac, le kétorolac, le méloxicam, le piroxicam, le ténoxicam, le naproxène, l'indométacine, le naproxcinod, le nimésulid, le célécoxib, l'étoricoxib, le parécoxib, le rofécoxib, le valdécoxib, la phénylbutazone, l'acide niflumique, l'acide méfénamique;

Ces agents actifs pourront être utilisés en une quantité de l'ordre de 0,01 à 20 % en poids, de préférence de 1 à 15 % en poids, et de préférence encore de 2 à 10 % en poids, rapportée au poids total de la composition.

La présence d'hydrocolloïdes au sein de la composition favorisera le relargage de ces agents actifs.

Bien entendu, la composition selon l'invention peut aussi comprendre un ou plusieurs autres composés connus pour leur action dans la phase de détersion comme par exemple :
- des enzymes ;
- l'urée.

### Adjuvants

A titre d'adjuvants susceptibles d'être utilisés dans les compositions selon l'invention, on peut citer des composés connus pour favoriser le relargage des agents actifs, comme par exemple les produits Montanox^{®} 80 ou Sepinov^{®} EMT 10 qui sont couramment utilisés dans les produits URGOTUL^{®} qui incorporent des agents actifs.

Ces adjuvants pourront être utilisés en une quantité de l'ordre de 1 à 15% en poids, rapportée au poids total de la composition.

Bien évidemment les modes de réalisation particuliers qui viennent d'être décrits peuvent être mis en œuvre séparément ou selon l'une quelconque de leurs combinaisons.

Les compositions selon l'invention permettent en particulier de réaliser des pansements interface autoportés ou des pansements interface présentant une armature ou un support.

Dans le cadre de la réalisation d'un pansement interface, on préférera l'utilisation d'une composition qui comprend des composés (copolymères, huile minérale, vaseline, antioxydant et hydrocolloïdes) de même nature que, ou identiques à, ceux utilisés dans le produit URGOTUL^{®}.

### Matrice élastomérique

Afin de réaliser un pansement, les compositions selon l'invention seront formées en couche mince, avec des trous traversants, disposés de préférence de façon répartie dans ladite couche pour former une matrice élastomérique.

L'invention a ainsi pour objet, selon un autre aspect, une matrice élastomérique obtenue à partir d'une composition selon l'invention telle que décrite précédemment.

Les trous traversants peuvent être réalisés par perforation ou poinçonnage d'une composition selon l'invention préalablement formée en couche mince, seule ou associée à un support provisoire ou à un film protecteur habituellement utilisé pour la fabrication de pansement, ou bien encore par une enduction tramée sur un support provisoire.

Alternativement, les matrices polymériques conformes à l'invention peuvent être fabriquées par coulage à chaud d'une composition telle que décrite précédemment sur une plaque gravée avec le motif retenu pour former des trous traversants, suivi d'un refroidissement et d'un démoulage.

D'une façon générale, les matrices polymériques conformes à l'invention présenteront une épaisseur comprise entre 0,4 mm et 2 mm, de préférence entre 0,5 mm et 1 mm, de préférence encore de l'ordre de 0,6 à 0,7 mm.

Les trous traversants peuvent être de géométrie quelconque et présenteront par exemple une section transversale circulaire, rectangulaire, trapézoïdale ou carrée.

Leur surface sera généralement comprise entre 1 et 7 mm².

Ces trous seront répartis, de préférence de façon régulière, avec une densité telle que la surface totale des trous représente entre 20 et 70 %, et de préférence entre 30 et 60 % de la surface totale du pansement.

Selon un mode de réalisation préféré, la matrice polymérique, lorsqu'elle est mise en œuvre dans un pansement interface, de préférence autoporté, se présente sous la forme d'un filet aéré (ou grille) de préférence de maille carrée présentant :
- une épaisseur du filet comprise entre 0,4 et 2 mm ;
- une « largeur de fil » (largeur de l'espace entre deux trous consécutifs) comprise entre 1 et 10 mm, et de préférence entre 1 et 5 mm ;
- un grammage compris entre 200 et 1700 g/m², et de préférence compris entre 300 et 800 g/m².

Selon un mode de réalisation particulièrement préféré de l'invention, une telle matrice élastomérique se présentera sous la forme d'un filet aéré à maille carrée présentant :
- une épaisseur du filet de 750 microns environ ;
- une largeur de fil (ou taille de maille) de l'ordre de 0,8 mm ;
- un grammage de l'ordre de 390 g/m².

Pour la réalisation de telles matrices élastomériques, on pourra se référer pour plus de détails à la demande de brevet FR 2 936 158.

Il peut également être envisagé d'utiliser cette matrice élastomérique pour enduire une armature ou un support.

Les techniques de fabrication d'un pansement interface avec armature ou avec support sont aussi bien connues de l'homme de l'art et on pourra par exemple se référer aux procédés décrits dans les demandes de brevet WO 00 16725 et FR 2 936 159 ou WO 2015/018720.

Selon un mode préféré de réalisation, les matrices élastomériques obtenues au moyen des compositions selon l'invention ne sont pas collantes, c'est-à-dire présentent un pouvoir adhésif sur peau, selon la méthode EN 1939, inférieur à 40 cN/cm, et préférence inférieur à 35 cN/cm. Un échantillon de support de 20 mm de large et 150 mm de long est posé sur l'avant-bras. Au bout de 10 minutes, on mesure le pouvoir adhésif avec un dynamomètre à une vitesse de traction de 900 mm/min avec un angle de 90°.

### Pansement

L'invention a ainsi pour objet, selon un mode préféré de réalisation, un pansement interface caractérisé en ce qu'il comprend une matrice élastomérique précédemment décrite.

Selon un mode de réalisation actuellement préféré, la présente demande vise à couvrir un pansement interface autoporté comprenant une matrice élastomérique se présentant sous la forme d'une couche mince possédant des trous traversants pour laisser passer les exsudats, obtenue à partir d'une composition comprenant :
- 5 à 20% d'un mélange de 2 copolymères tribloc du type styrène - oléfine saturée - styrène, un premier qui présente une viscosité comprise entre 0,01 et 1 Pa.s telle que mesurée dans une solution à 5 % (masse/masse) dans le toluène et un second qui présente une viscosité comprise entre 0 ,01 et 0,5 Pa.s mesurée dans une solution à 15 % (masse / masse) dans le toluène
- 50 à 80% en poids d'au moins un plastifiant,
- 5 à 20% d'au moins une résine de type alpha-méthyl styrène dont le point de ramollissement se situe entre 80 et 125°C, de préférence entre 90 et 110°C, étant entendu que :
   - lorsque la teneur en copolymères tribloc est comprise entre 5 et 10% en poids, la teneur en résine est comprise entre 15 et 20% en poids, rapporté au poids total de la composition et
   - lorsque la teneur en copolymères tribloc est supérieure à 10% en poids, la teneur en résine est comprise entre 5 et 20% en poids, rapporté au poids total de la composition,

les pourcentages étant rapportés au poids total de la composition.

De préférence, le pansement interface selon la présente invention n'adhère pas aux gants en latex. Pour ce faire, la composition peut de préférence comprendre :
- pour 100 parties en poids d'un mélange P de 2 copolymères tribloc spécifiques du type styrène - oléfine saturée - styrène, un premier qui présente une viscosité comprise entre 0,01 et 1 Pa.s telle que mesurée dans une solution à 5 % (masse/masse) dans le toluène et un second qui présente une viscosité comprise entre 0 ,01 et 0,5 Pa.s telle que mesurée dans une solution à 15 % (masse/masse) dans le toluène ;
- de 300 à 1000 parties en poids d'un plastifiant H, de préférence une huile plastifiante ; et
- de 90 à 600 parties en poids de vaseline V ; étant en outre précisé que :
   - la quantité totale, représentée par P+H+V, de mélange d'élastomères, du plastifiant et de la vaseline est comprise entre 490 et 1700 parties en poids ;
   - le rapport entre la quantité totale du mélange d'élastomères, du plastifiant et de la vaseline et la quantité de vaseline, représenté par P+H+V/V, est inférieur à 11;
      ledit mélange de 2 copolymères comprend au moins 20 % en poids du premier copolymère,
      la composition comprenant en outre de 5 à 20 % en poids d'une résine de type alpha-méthyl styrène dont le point de ramollissement se situe entre 80 et 125°C, de préférence entre 90 et 110°C,
      étant entendu que :
         - lorsque la teneur en copolymères tribloc est comprise entre 5 et 10% en poids, la teneur en résine est comprise entre 15 et 20% en poids, rapporté au poids total de la composition et
         - lorsque la teneur en copolymères tribloc est supérieure à 10% en poids, la teneur en résine est comprise entre 5 et 20% en poids, rapporté au poids total de la composition,

les pourcentages étant rapportés au poids total de la composition.

Afin de protéger la composition de l'environnement extérieur, le pansement interface pourra être recouvert, de préférence sur chacune de ses faces, par un film protecteur provisoire qui sera retiré avant usage par l'utilisateur.

Afin de faciliter encore la manipulation du pansement interface, en particulier s'il est autoporté, on pourra substituer ces deux films protecteurs provisoires par un protecteur unique tel que décrit dans la demande de brevet WO 2008 / 145 884 ou dans la demande de brevet WO2015/018720 dont la structure particulière facilite l'application du pansement sur la plaie.

La présente invention est illustrée dans les exemples non limitatifs présentés ci-après.

### EXEMPLES

### Préparation des compositions

Les compositions des exemples 1 à 26 ont été élaborées à l'aide des constituants suivants dans les proportions, exprimées en pourcentage en poids, mentionnées dans le tableau 1 ci-dessous.
Elastomère : copolymère séquencé de poly (styrène-éthylène-butylène-styrène) (en abrégé SEBS) :
   - KRATON^{®} G 1654 ES viscosité à 5% (masse/masse) dans le toluène : 0,02Pa.s
   - KRATON^{®} G 1650 E vicosité à 15% (masse / masse) dans le toluène : 0,2Pa.s
Plastifiant : huile minérale Ondina^{®} 919 commercialisée par la société SHELL ou Pionier 2076P commercialisée par Hansen & Rosenthal
Vaseline : vaseline Codex^{®} A commercialisée par la société AIGLON
Antioxydant : IRGANOX^{®} 1010 commercialisé par la société BASF
Hydrocolloïde : Carboxyméthylcellulose sodique CMC BLANOSE^{®} 7H4XF commercialisée par la société ASHLAND,

### Résines :

- Wingtack 86, résine hydrocarbonnée C9 modifiée C5, présentant un point de ramollissement situé entre 84-90°C, commercialisée par Cray Valley,
- Norsolène W90 ou Cleartack W90, résine poly(styrène-co-alpha-méthyl styrène) présentant un point de ramollissement situé entre 85-95°C, commercialisée par Cray Valley,
- Norsolène W140, résine poly(styrène-co-alpha-méthyl styrène) présentant un point de ramollissement situé entre 135-145°C, commercialisées par Cray Valley,
- Escorez 5380, résine hydrocarbonée cycloaliphatique, présentant un point de ramollissement situé entre 80-90°C, commercialisée par Exxon Mobil,
- YS Resin SX 100, résine polystyrène, présentant un point de ramollissement situé à 100°C, commercialisée par YASUHARA CHEMICAL,
- Sylvares SA 100, résine alpha-méthyl styrène présentant un point de ramollissement situé entre 95 et 105°C, commercialisée par Arizona Chemical/Kraton Polymer
- Sylvares SA 120 alpha-méthyl styrène présentant un point de ramollissement situé entre 115 et 125°C, commercialisée par Arizona Chemical/Kraton Polymer
- Kristalex 3100LV, résine poly(styrène-co-alpha-méthyl styrène) présentant un point de ramollissement de 100°C, commercialisée par Eastman,
- Kristalex F100 poly(styrène-co-alpha-méthyl styrène) présentant un point de ramollissement situé entre 96 et 104°C, commercialisée par Eastman,
- Kristalex F115, résine poly(styrene-co-alpha-methylstyrene) présentant un point de ramollissement situé entre 114 et 120°C, commercialisée par Eastman,
- Kristalex 5140, résine poly(styrene-co-alpha-methylstyrene) présentant un point de ramollissement de 139°C, commercialisée par Eastman,
- Sukorez SU-400, résine polycyclopentadiene présentant un point de ramollissement situé entre 97 et 107°C, commercialisé par Kolon Industries.

### Fabrication de la composition

Dans un mélangeur vertical, on a introduit successivement à une température de consigne de 90°C le plastifiant, l'hydrocolloïde et la vaseline et on a agité jusqu'à l'obtention d'un mélange homogène.

On a ensuite introduit sous agitation le(s) copolymère(s), l'antioxydant, ainsi que la (les) résines puis on a porté la température de consigne à 150°C et agité jusqu'à l'obtention d'un mélange homogène. On a ensuite placé le mélangeur sous vide pour supprimer les bulles présentes dans le mélange.

On a ensuite laissé refroidir, puis on a vidangé le mélangeur.

Par la suite, on a réalisé des matrices polymériques à partir des compositions à tester, en appliquant une forte pression à l'aide d'une presse hydraulique selon le protocole suivant :
On a préchauffé les 2 plateaux de la presse hydraulique. Sur le plateau inférieur de la presse on a déposé un film plastique anti-adhérent, par exemple un film de polyester siliconé - fluoré (la face siliconée - fluorée étant disposée de façon opposée au plateau inférieur). On a déposé sur cette face environ 12 g d'une des compositions décrites et on a recouvert cette dernière par un film de polyester siliconé (le côté siliconé étant disposé au contact de la composition). On a placé 2 cales de 0,75 mm entre les 2 films de polyester aux extrémités du plateau inférieur de la presse et on a soumis l'ensemble à une pression de 200 bars et une température de l'ordre de 90 à 100 C°.

On a laissé refroidir les plaques ainsi réalisées et on a contrôlé leurs épaisseurs avec un micromètre de manière à obtenir une maquette dont l'épaisseur est de l'ordre de 650µm.

### Mesure de la force à la rupture et de l'allongement à la rupture :

Les conditions de réalisation du test et des maquettes utilisées étaient les suivantes.

Le principe de cette mesure est d'exercer une traction sur une éprouvette haltère (correspondant à une trame élastomérique emporte-piécée) à vitesse constante V jusqu'à la rupture à l'aide d'un dynamomètre.

On mesure ainsi la force à la rupture et l'allongement à la rupture.

### MATERIEL / APPAREILLAGE

Presse automatique + cales d'épaisseur,
Dynamomètre électronique (0,1 à 999 mm/min),
Capteur de force (à adapter selon force mesurée),
Emporte-pièce éprouvette haltère,
Matériau type mousse polyuréthane mécanique 400 µm Exopack.

### ECHANTILLONNAGE/ CONDITIONNEMENT DES ECHANTILLONS

Nombre d'éprouvette n ≥ 5.
Temps de conditionnement T > 24h.
Température T = 23°C ± 2 °C.
Hygrométrie HR = 50% ± 15 %.

### CONDITIONS OPERATOIRES

- l (largeur de l'éprouvette) = 12,7 mm
- l₀ (distance entre les mors du dynamomètre) =90 mm
- V (vitesse de traction) = 300 mm/min

### MODE OPERATOIRE

Préparation des éprouvettes :
- Débuller la composition à analyser au préalable,
- A l'aide de la presse automatique, préparer des plaques de matrice élastomérique de 650 µm d'épaisseur à partir de la composition débullée (cales de 750 µm),
- Découper les éprouvettes haltères à l'aide de l'emporte-pièce, en veillant à ne pas faire d'amorce.

### MESURE

- Placer une éprouvette haltère de largeur 12,7 mm dans les mors du dynamomètre distants de l₀ = 90 mm, en recouvrant préalablement les 2 extrémités de mousse PU mécanique 400 µm (pour éviter le cisaillement de l'éprouvette au niveau des mors),
- Procéder à l'essai de traction jusqu'à la rupture de l'éprouvette à la vitesse V = 300 mm/min,
- Vérifier que le front de rupture se produit dans la partie rectiligne de l'éprouvette.

### CALCULS / EXPRESSION DES RESULTATS

- Extraire de la courbe les valeurs de force à la rupture et d'allongement à la rupture associées.
- Calculer pour chaque référence d'échantillon :
- le minimum,
- le maximum,
- la moyenne,
- l'écart-type,
- le CV.

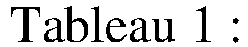

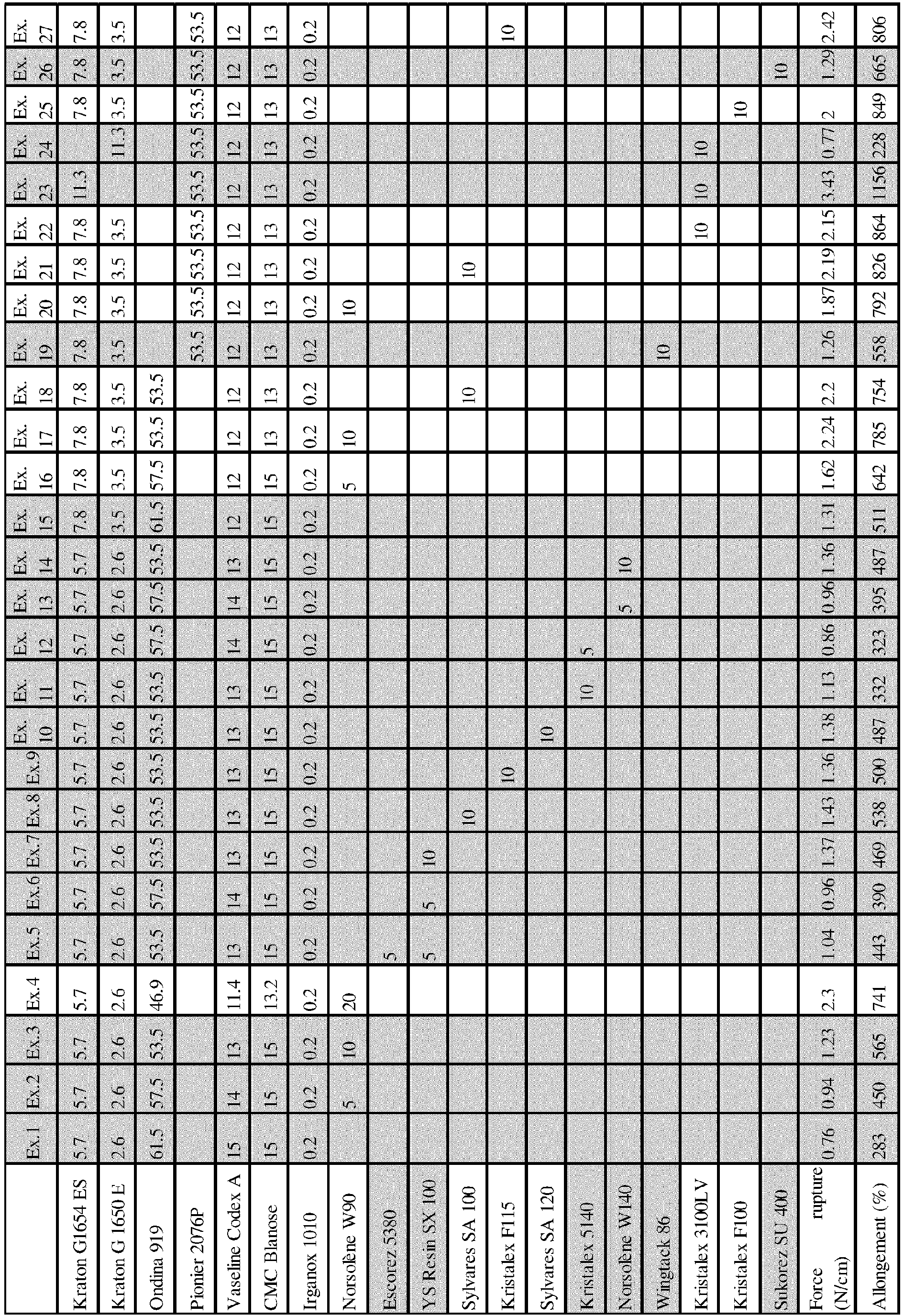

La matrice obtenue aux exemples 1 et 15 ne contenant pas de résine, présente de mauvais résultats de force et d'allongement à la rupture.

La matrice obtenue aux exemples 2 et 3 (et celles des exemples 5 à 14) contient une teneur totale en copolymères triblocs inférieure à 10% en poids et une teneur en résine inférieure à 15%, ce qui ne permet pas d'obtenir de bons résultats de force et d'allongement à la rupture. Au contraire, la matrice obtenue à l'exemple 4 selon l'invention, comprenant une teneur totale en copolymères triblocs inférieure à 10% en poids et en résine de 20%, présente bons résultats de force et d'allongement à la rupture, ce qui se traduit par de bonnes propriétés mécaniques, en particulier de résistance à la déchirure.

En outre, les matrices élastomériques obtenues aux exemples 5, 6, 7, 11, 12, 13 et 14 mettant en œuvre des résines non conforme à la présente invention ne donnent pas de bons résultats de force et d'allongement à la rupture.

Les matrices élastomériques obtenues aux exemples 5, 6, 7, 11 et 12 présentent en outre une couleur blanchâtre caractéristique d'une instabilité du mélange, et déceptif d'un point de vue esthétique.

Les matrices élastomériques obtenues aux exemples 16, 17, 18, 20, 21, 22, 25 et 27 conformes à l'invention présentent bons résultats de force et d'allongement à la rupture, ce qui se traduit par de bonnes propriétés mécaniques, en particulier de résistance à la déchirure.

La matrice élastomérique obtenue aux exemples 19 et 26, mettant en œuvre des résines non conformes à la présente invention, ne présente pas de bons résultats force et d'allongement à la rupture.

Les matrices élastomériques des exemples 23 et 24, ne contenant qu'un seul copolymère tribloc selon l'invention, ne donnent pas non plus satisfaction en terme de résistance à la déchirure.

## Revendications

1. Composition comprenant:
- 5 à 20% d'un mélange de 2 copolymères tribloc du type styrène - oléfine saturée - styrène, un premier qui présente une viscosité comprise entre 0,01 et 1 Pa.s telle que mesurée à 30°C dans une solution à 5 % (masse/masse) dans le toluène et un second qui présente une viscosité comprise entre 0 ,01 et 0,5 Pa.s mesurée à 30°C dans une solution à 15 % (masse / masse) dans le toluène
- 50 à 80% en poids d'au moins un plastifiant,
- 5 à 20% d'au moins une résine de type alpha-méthyl styrène dont le point de ramollissement (mesuré selon la norme ISO 4625, méthode "Ring and Ball") se situe entre 80 et 125°C, de préférence entre 90 et 110°C,
étant entendu que :
- lorsque la teneur en copolymères tribloc est comprise entre 5 et 10% en poids, la teneur en résine est comprise entre 15 et 20% en poids, rapporté au poids total de la composition et
- lorsque la teneur en copolymères tribloc est supérieure à 10% en poids, la teneur en résine est comprise entre 5 et 20% en poids, rapporté au poids total de la composition,
les pourcentages étant rapportés au poids total de la composition.

2. Composition selon la revendication 1, **caractérisée en ce que** la résine est choisie parmi une résine alpha-méthyl styrène présentant un point de ramollissement situé entre 95 et 105°C ou entre 115 et 125°C ou une résine poly(styrène-co-alpha-méthyl styrène) présentant un point de ramollissement entre 95°C et 115°C.

3. Composition selon l'une des revendications 1 ou 2, **caractérisée en ce que** le plastifiant est constitué d'un mélange d'huile minérale et de vaseline.

4. Composition selon l'une des revendications précédentes, **caractérisée en ce qu'**elle comprend des particules d'hydrocolloïde en une quantité inférieure ou égale à 25 % en poids, rapportée au poids total de la composition.

5. Composition selon l'une des revendications précédentes, **caractérisée en ce qu'**elle comprend une ou plusieurs substances active(s) permettant d'induire ou d'accélérer la cicatrisation ou pouvant avoir un rôle favorable dans le traitement des plaies, en une quantité comprise entre 0,01 et 20 % en poids, de préférence entre 1 et 15 % en poids, rapportée au poids total de la composition.

6. Matrice élastomérique, **caractérisée en ce qu'**elle est obtenue à partir d'une composition selon l'une des revendications 1 à 5, de préférence par formation d'une couche mince et compression, ou par coulage à chaud de ladite composition.

7. Matrice élastomérique selon la revendication 6, **caractérisée en ce qu'**elle comprend une armature ou un support, ou **en ce qu'**elle est autoportée.

8. Matrice élastomérique autoportée selon la revendication 7, **caractérisée en ce qu'**elle comporte des trous traversants.

9. Matrice élastomérique autoportée selon l'une des revendications 7 ou 8, **caractérisée en ce qu'**elle se présente sous la forme d'un filet aéré dont la taille de maille est de l'ordre de 4mm², l'épaisseur de l'ordre de 800 microns et le grammage de l'ordre de 400 g/m².

10. Pansement interface, **caractérisé en ce qu'**il comprend une matrice élastomérique selon l'une des revendications 6 à 9.

## Patentansprüche

1. Zusammensetzung, umfassend:
- 5 bis 20 % einer Mischung von 2 Triblock-Copolymeren vom Typ Styrol - gesättigtes Olefin - Styrol, ein erstes, das eine Viskosität von mindestens 0,01 und nicht mehr als 1 Pa·s hat, gemessen bei 30 °C in einer Lösung von 5 % (Masse/Masse) in Toluol, und ein zweites, das eine Viskosität von mindestens 0,01 und nicht mehr als 0,5 Pa·s hat, gemessen bei 30°C in einer Lösung von 15 % (Masse/Masse) in Toluol,
- 50 bis 80 Gew.-% mindestens eines Weichmachers,
- 5 bis 20 % mindestens eines Harzes vom α-Methylstyrol-Typ, dessen Erweichungspunkt zwischen 80 und 125°C, vorzugsweise zwischen 90 und 110°C liegt, gemessen nach der ISO-Norm 4625 gemäß der "Ring-und-Kugel"-Methode, wobei gilt:
- wenn der Gehalt an Triblock-Copolymeren zwischen 5 und 10 Gew.-% liegt, beträgt der Harzgehalt zwischen 15 und 20 Gew.-%, bezogen auf das Gesamtgewicht der Zusammensetzung, und
- wenn der Gehalt an Triblock-Copolymeren größer als 10 Gew.-% ist, beträgt der Harzgehalt zwischen 5 und 20 Gew.-%, bezogen auf das Gesamtgewicht der Zusammensetzung,
wobei die Prozentsätze sich auf das Gesamtgewicht der Zusammensetzung beziehen.

2. Zusammensetzung nach Anspruch 1, **dadurch gekennzeichnet, dass** das Harz ausgewählt ist aus einem α-Methylstyrol-Harz mit einem Erweichungspunkt zwischen 95 und 105 °C oder zwischen 115 und 125 °C oder einem Poly-(styrol-co-α-methylstyrol)-Harz mit einem Erweichungspunkt zwischen 95 °C und 115 °C.

3. Zusammensetzung nach einem der Ansprüche 1 oder 2, **dadurch gekennzeichnet, dass** der Weichmacher aus einer Mischung von Mineralöl und Petrolatum besteht.

4. Zusammensetzung nach einem der vorhergehenden Ansprüche, **dadurch gekennzeichnet, dass** sie Hydrokolloidpartikel in einer Menge von kleiner oder gleich 25 Gew.-%, bezogen auf das Gesamtgewicht der Zusammensetzung, enthält.

5. Zusammensetzung nach einem der vorhergehenden Ansprüche, **dadurch gekennzeichnet, dass** sie einen Wirkstoff oder mehrere Wirkstoffe enthält, der/die es ermöglicht/ermöglichen die Wundheilung zu veranlassen oder zu beschleunigen, oder der/die eine günstige Rolle bei der Behandlung von Wunden spielen kann/können, in einer Menge von mindestens 0,01 und nicht mehr als 20 Gew.-%, vorzugsweise von mindestens 1 und nicht mehr 15 Gew.-%, bezogen auf das Gesamtgewicht der Zusammensetzung.

6. Elastomermatrix, **dadurch gekennzeichnet, dass** sie aus einer Zusammensetzung nach einem der Ansprüche 1 bis 5 erhalten wird, vorzugsweise durch Ausbilden einer dünnen Schicht und Verpressen oder durch Heißgießen dieser Zusammensetzung.

7. Elastomermatrix nach Anspruch 6, **dadurch gekennzeichnet, dass** sie einen Rahmen oder eine Stütze umfasst oder selbsttragend ist.

8. Selbsttragende Elastomermatrix nach Anspruch 7, **dadurch gekennzeichnet, dass** sie Durchgangslöcher umfasst.

9. Selbsttragende Elastomermatrix nach einem der Ansprüche 7 oder 8, **dadurch gekennzeichnet, dass** sie in Form eines belüfteten Netzes vorliegt, dessen Maschenweite in der Größenordnung von 4 mm² liegt, dessen Dicke in der Größenordnung von 800 Mikrometer liegt, und dessen Flächengewicht in der Größenordnung von 400 g/m² liegt.

10. Auflage-Pflaster, **dadurch gekennzeichnet, dass** es eine Elastomermatrix nach einem der Ansprüche 6 bis 9 umfasst.

## Claims

1. Composition comprising:
- 5 to 20% of a mixture of two triblock copolymers of the type styrene-saturated olefin-styrene, a first which has a viscosity between 0.01 and 1 Pa.s as measured in a 5% (mass/mass) solution in toluene and a second which has a viscosity between 0.01 and 0.5 Pa.s as measured in a 15% (mass/mass) solution in toluene,
- 50 to 80% by weight of at least one plasticiser,
- 5 to 20% of at least one alpha-methylstyrene resin, having a softening point, measured according to standard ISO 4625 "Ring and Ball" method, in the range between 80 and 125°C, preferably between 90 and 110°C,
it being understood that:
- when the content of triblock copolymers is between 5 and 10% by weight, the content of resin is between 15 and 20% by weight, with respect to the total weight of the composition, and
- when the content of triblock copolymers is greater than 10% by weight, the content of resin is between 5 and 20% by weight, with respect to the total weight of the composition, and
the percentages being with respect to the total weight of the composition.

2. Composition according to claim 1, **characterised in that** the resin is chosen from an alpha-methylstyrene resin having a softening point in the range between 95 and 105°C or between 115 and 125°C or a poly(styrene-co-alpha-methylstyrene) resin having a softening point between 95°C and 115°C.

3. Composition according to one of claims 1 or 2, **characterised in that** the plasticiser is composed of a mixture of mineral oil and vaseline.

4. The composition according to one of the preceding claims, **characterised in that** it comprises hydrocolloid particles in a quantity less than or equal to 25% by weight, with respect to the total weight of the composition.

5. Composition according to one of the preceding claims, **characterised in that** it comprises one or more active substances for inducing or accelerating healing or able to play a beneficial role in the treatment of wounds, in a quantity between 0.01 and 20% by weight, preferably between 1 and 15% by weight, with respect to the total weight of the composition.

6. Elastomeric matrix, **characterised in that** it is obtained from a composition according to one of claims 1 to 5, preferably by formation of a thin layer and compression, or by hot casting of said composition.

7. Elastomeric matrix according to claim 6, **characterised in that** it comprises a framework or a substrate, or **in that** it is self-supporting.

8. Self-supporting elastomeric matrix according to claim 7, **characterised in that** it comprises through-holes.

9. Self-supporting elastomeric matrix according to claims 7 or 8, **characterised in that** it is in the shape of an aerated net with a mesh size of around 4 mm², thickness of around 800 microns and grammage of around 400 g/m².

10. Interface dressing, **characterised in that** it comprises an elastomeric matrix according to one of claims 6 to 9.
